# EUROPEAN PATENT SPECIFICATION

(11) **EP 2 449 953 B1**
(45) Date of publication and mention of the grant of the patent: **03.09.2014**
(21) Application number: 11250796.7
(22) Date of filing: 14.09.2011
(51) Int. Cl.: A61B 1/00

(54) **Mirrored arthroscope**
Gespiegeltes Arthroskop
Arthroscope à miroir

(30) Priority: 03.11.2010 US 409574 P; 07.09.2011 US 226516
(43) Date of publication of application: 09.05.2012
(73) Proprietor: Covidien LP, Mansfield, MA 02048 (US)
(72) Inventor: Sargeant, Timothy, Guilford, CT 06437 (US); Desai, Arpan, Hamden, CT 06514 (US); Agawu, Atu, Princeton, NJ 08540 (US)
(74) Representative: Soames, Candida Jane

(56) References cited:
- DE-A1- 19 943 080
- JP-A- 62 133 415
- US-A1- 2004 017 626
- US-A1- 2006 106 286

## Description

### CROSS REFERENCE TO RELATED APPLICATION

### BACKGROUND

### Technical Field

The present disclosure relates to a surgical instrument, and more particularly, to a mirrored arthroscopic camera for providing better visualization within the joint space.

### Background of Related Art

Endoscopic surgical procedures are minimally invasive procedures in which operations are carried out within the body by using elongated instruments inserted through small entrance openings in the body. Arthroscopic surgical procedures, a subset of endoscopic surgical procedures, are minimally invasive procedures carried out within the interior of a joint, i.e., the joint space.

Minimally invasive procedures, e.g., arthroscopic procedures, are desirable in that they allow for quicker recovery time and shorter hospital stays as compared to open surgical procedures. Minimally invasive procedures also leave minimal scarring (both internally and externally) and reduce patient discomfort during the recovery period. However, because the interior dimensions of the entrance openings into the body are necessarily small, only elongated, small diametered instrumentation may be used to access the internal joint space.

During an arthroscopic procedure, for example, an arthroscope, an elongated tubular instrument that allows a surgeon to illuminate and view the joint space, is inserted into the interior of the joint through a small incision. As can be appreciated, the maneuverability of the arthroscope is limited by the dimensions of the joint space as well as by the dimensions of the entrance opening into the body. Thus, due to the spatial constraints of arthroscopic procedures, the ability to maneuver the arthroscope within the interior of the joint to illuminate and view the joint space is limited.

US 2006/0106286 discloses an endoscope with a mirror fixed at a distal end and US 2004/0017626 discloses a mirror attached to the distal end of a medical instrument which is deployed by extension of an arm to which it is mounted.

### SUMMARY

In accordance with one embodiment of the present disclosure, a surgical instrument is provided as recited in claim 1.

In one embodiment, the light source is a fiber optic bundle extending through the elongated tubular member. An illuminated distal tip of the fiber optic bundle is configured to emit light distally from the distal end of the elongated tubular member. The fiber optic cables capture and transmit an image from the distal end of the elongated tubular member to a remote video display for displaying the image as a video image.

In another embodiment, the light source is a light emitting diode (LED). In such an embodiment, a camera may be disposed within the elongated tubular member and positioned at the distal end thereof. The camera is configured to capture and transmit an image from the distal end of the elongated tubular member to a remote video display for displaying the image as a video image.

In yet another embodiment, the mirror assembly includes an arm, a base, and a mirror. The arm is fixedly engaged to the base at one end thereof and extends therefrom. The mirror is hingeably engaged to the arm at the other end of the arm such that the mirror may be angled with respect to the arm to re-direct light radially off the longitudinal axis of the elongated tubular member. The mirror may be angled to re-direct light at an angle of about zero degrees to about 90 degrees with respect to the longitudinal axis in each of the distal and proximal directions. Additionally, the mirror of the mirror assembly may define a convex, concave, or substantially flat mirrored surface.

The hinging (or angling) of the mirror with respect to the arm may be either mechanically or electrically controlled. Further, the mirror assembly may be integral with the elongated tubular member, or may be releasably coupled to the elongated tubular member.

In another embodiment, the mirror assembly is longitudinally translatable, e.g., between the retracted and the extended position, along a track disposed on an outer surface of the elongated tubular member. Translation of the mirror assembly between the retracted position and the extended position may be either mechanically or electrically controlled.

In still yet another embodiment, the elongated tubular member is formed from a rigid or a semi-rigid material.

In accordance with another aspect of the present disclosure, the mirror assembly includes a base that is releasably engageable with an outer surface of an arthroscopic camera. An arm is fixedly engaged at a first end thereof to the base and is hingeably coupled to a mirror at a second end thereof. The arm is configured to extend beyond the distal end of the arthroscopic camera. The mirror is hingeable with respect to the arm, and thus, with respect to the arthroscopic camera, to re-direct light to/from a distal end of the camera to define a viewing direction of the camera.

In one embodiment, the mirror is hingeable with respect to the arthroscopic camera to define the viewing direction of the camera off a longitudinal axis of camera at an angle of about zero degrees to about 90 degrees with respect to the longitudinal axis in each of the distal and proximal directions.

In yet another embodiment, the arm is moveable between a retracted position and an extended position. In the retracted position, the mirror is positioned adjacent the distal end of the arthroscopic camera. In the extended position, the mirror is extended beyond a distal end of the arthroscopic camera.

In another embodiment, a control wire (or control wires) is coupled to the arm at a first end thereof. The control wire is configured to extend along (or through) the arthroscopic camera to ultimately couple to a control member for remotely controlling the hinging of the mirror with respect to the arm. Translation of the arm from the retracted position to the extended position may also be remotely controlled by the control member (via the control wire). Further, the hinging of the mirror with respect to the arm and/or the extension/retraction of the arm may be electrically or mechanically controlled.

### BRIEF DESCRIPTION OF THE DRAWINGS

Various embodiments of the subject instrument are described herein with reference to the drawings wherein:

Fig. 1 is a side, cross-sectional view of an arthroscopic camera including a mirror assembly in accordance with the present disclosure, showing the mirror assembly in a retracted position;

Fig. 2 is a side, cross-sectional view of the arthroscopic camera of Fig. 1, showing the mirror assembly in an extended position;

Fig. 3 is a perspective view of the arthroscopic camera of Fig. 1, showing a mirror of the mirror assembly angled with respect to an arm of the mirror assembly in a first position;

Fig. 4 is a perspective view of the arthroscopic camera of Fig. 1, showing the mirror angled with respect to the arm in a second position;

Fig. 5 is a side, cross-sectional view of the arthroscopic camera of Fig. 1, shown disposed through an opening in tissue with the mirror disposed in one position; and

Fig. 6 is a side, cross-sectional view of the arthroscopic camera of Fig. 1, shown disposed through an opening in tissue with the mirror disposed in another position.

### DETAILED DESCRIPTION

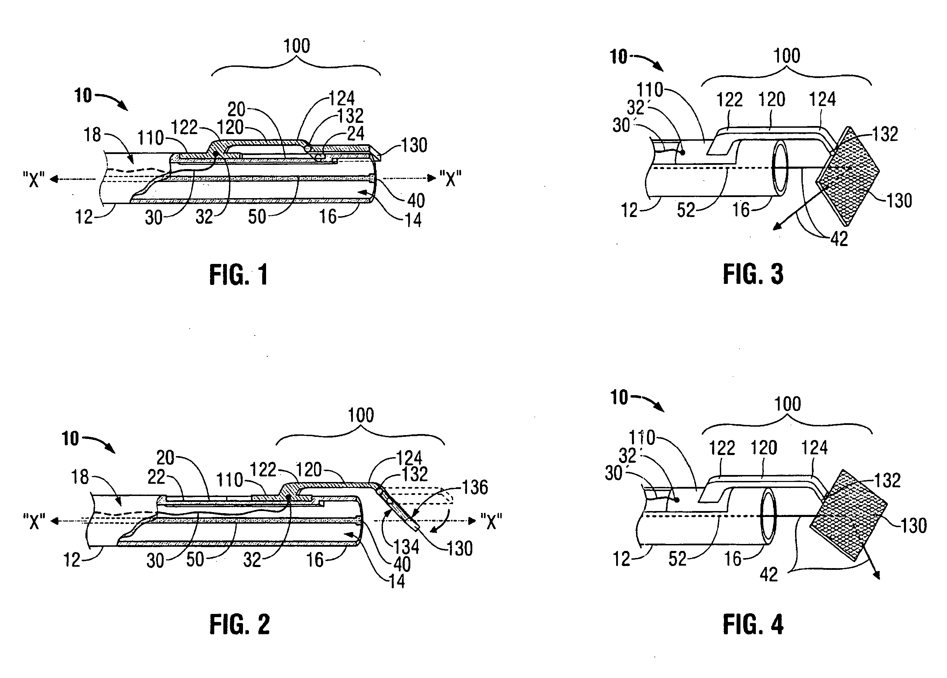

Referring now to Figs. 1-4, a surgical camera for use in endoscopic and, more particularly, in arthroscopic surgical procedures is shown generally designated by reference numeral 10. Arthroscopic camera, or arthroscope 10 includes an elongated tubular member 12 defining a longitudinal axis "X" and has a lumen 14 extending therethrough. Elongated tubular member 12 is configured to house the internal working components of arthroscope 10 therein. A mirror assembly 100 is coupled to a distal end 16 of elongated tubular member 12 on an outer surface 18 thereof and is moveable with respect to elongated tubular member 12 between a retracted position and an extended position.

It is envisioned that elongated tubular member 12 be made from a rigid, or semi-rigid material to protect the internal components of arthroscope 10 and to facilitate insertion of arthroscope 10 into a surgical site, e.g., the joint space. It is also envisioned that elongated tubular member 12 and/or mirror assembly 100 be formed form a biocompatible material to reduce the incidence of adverse reaction by a patient upon contact with the patient's tissue.

As shown in Figs. 1-4, mirror assembly 100 includes a base 110, an arm 120, and a mirror 130. The mirror assembly 100 is longitudinally translatable with respect to elongated tubular member 12 along a track 20 disposed on and extending longitudinally along outer surface 18 of elongated tubular member 12 towards distal end 16 thereof. More specifically, base 110 is engaged within track 20 and is translatable along track 20 to move mirror assembly 100 between the retracted position, wherein base 110 of mirror assembly 100 is positioned at a proximal end 22 of track 20 such that mirror 130 is positioned adjacent outer surface 18 of elongated tubular member 12 (Fig. 1), and the extended position, wherein base 110 of mirror assembly 100 is translated to a distal end 24 of track 20 such that mirror 130 extends distally from distal end 16 of elongated tubular member 12 (Fig. 2).

With continued reference to Figs. 1-4, arm 120 is fixedly engaged to base 110 at a first end 122 thereof and is hingeably coupled to mirror 130 at a second end 124 thereof. Mirror 130 may be hingeably coupled to arm 120 in any suitable fashion, e.g., a pin-aperture engagement 132, such that mirror 130 may be angled with respect to arm 120. It is envisioned that mirror 130 be angleable with respect to arm 120 from about zero (0) degrees, i.e., where mirror 130 is substantially parallel with arm 120, to at least about 90 degrees, i.e., where mirror 130 is substantially perpendicular to arm 120, however it is also contemplated that mirror 130 be angleable with respect to arm 120 up to about 180 degrees. It is also contemplated that mirror 130 be angleable with respect to arm 120 via a ball and socket mechanism (not shown), to permit mirror 130 to be angled in even more directions with respect to arm 120.

Mirror 130 includes a mirrored surface 134 and a back surface 136 and is configured such that, when mirror assembly 100 is translated to the extended position, mirrored surface 134 extends distally beyond elongated tubular member 12. As can be appreciated, angling mirror 130 with respect to arm 120 similarly angles mirrored surface 134 of mirror 130 with respect to elongated tubular member 12, re-directing light emitted from distal end 16 of elongated tubular member 12, as will be discussed in greater detail below and as shown in Figs. 3 and 4. Further, mirrored surface 134 of mirror 130 may be concave, convex, substantially flat, or otherwise configured to facilitate the re-direction of light to/from distal end 16 of elongated tubular member 12.

As shown in Figs. 1-2, a control wire 30, or control wires 30, may extend through elongated tubular member 12, coupling to mirror assembly 100 at a first end 32 of wire(s) 30 for remotely controlling the translation of mirror assembly 100 between the retracted position and the extended position and/or for angling mirror 130 with respect arm 120. Accordingly, a second end (not shown) of control wire(s) 30 may be coupled to a control member (not shown) for mechanically or electrically controlling the position of mirror assembly 100 and/or mirror 130. For example, a joystick, or other control member (not shown) may be used to selectively tension control wire(s) 30 to mechanically position mirror assembly 100 and/or mirror 130. Alternatively, a trigger, or switch (not shown) may be provided to, upon actuation, transmit an electrical signal through the control wires 30 to selectively position mirror assembly 100 and/or mirror 130. Further, the control member (not shown) may be wireless, thus being capable of wirelessly communicating with an antenna (not shown) disposed at a proximal end of elongated tubular member 12 and coupled to control wire(s) 30 for electrically controlling the movement of mirror assembly 100 and/or mirror 130.

In some embodiments, mirror assembly 100 may be releasably mountable on an arthroscopic camera, e.g., arthroscope 10. In such embodiments, first end 122 of arm 120 may be engaged within and translatable along a track (not shown) extending longitudinally along base 110, as opposed to base 110 translating along track 20 defined within elongated tubular member 12. In operation, the releasable mirror assembly (not shown) functions substantially similarly to fixed, or integrated mirror assembly 100 discussed above, the main difference being that the releasable mirror assembly (not shown) is releasably coupled to an arthroscopic, or endoscopic camera, e.g., arthroscope 10, while mirror assembly 100 is integral with arthroscope 10. Thus, the releasable mirror assembly (not shown) is similarly moveable between a retracted position and an extended position. In the retracted position, first end 122 of arm 120 is disposed at the proximal end of the track defined within base 110 and, correspondingly, arm 120 and mirror 130 are positioned adjacent elongated tubular member 12. In the extended position, wherein first end 122 of arm 120 has been translated to the distal end of the track defined within base 110, at least a portion of arm 120, and thus mirror 130, extends distally from distal end 16 of elongated tubular member 12.

Additionally, in embodiments where the mirror assembly is releasably mountable on arthroscope 10, base 110 may be configured to snap-fit, clip-on, or otherwise releasably engage outer surface 18 of elongated tubular member 12 of arthroscope 10. Further, base 110 may be dimensioned to engage a specific arthroscope, e.g., arthroscope 10, a specific configuration of arthroscope, or, alternatively, may be adjustable to engage arthroscopes of varying diameter and/or configuration.

Similarly as discussed above in relation to integrated mirror assembly 100, the translation, or extension, of the detachable mirror assembly (not explicitly shown) between the retracted position and the extended position may be remotely controlled. Accordingly, an electrical lead, or wire 30' (Figs. 3-4) may be coupled to the detachable mirror assembly. It is envisioned that wire 30' (Figs. 3-4) have sufficient length to extend along outer surface 18 of arthroscope 10 to communicate with a remotely positioned control member (not shown). Wire 30' (Figs. 3-4) may be clipped or otherwise releasably securable to elongated tubular member 12, to prevent catching on tissue or otherwise interfering with the insertion and/or removal of arthroscope 10 from an opening in tissue. Alternatively, the detachable mirror assembly may include an antenna (not shown) disposed thereon and configured for wireless communication with the remotely positioned control member (not shown). Thus, in either embodiment, the position of mirror assembly 100 and/or mirror 130 of the detachable mirror assembly (not shown) may be controlled from a remotely positioned control member (not shown).

With continued reference to Figs. 1-2, arthroscope 10, as mentioned above, includes an elongated tubular member 12 having a lumen 14 extending therethrough. A light source 40 is positioned within lumen 14 of elongated tubular member 12 at distal end 16 thereof. Light source 40 is configured to emit light (schematically indicated by arrow 42), or illuminate the field of view of arthroscope 10. More specifically, light source 40 is configured to direct light 42 from distal end 16 of elongated tubular member 12 distally along the longitudinal axis "X." Light source 40 may be an illuminated tip of a fiber optic bundle, a light emitting diode (LED), or any other suitable light emitting mechanism.

A transmitting component 50, e.g., a camera, or the fiber optic bundle, is coupled to distal end 16 of elongated tubular member 12 and extends proximally through lumen 14 of elongated tubular member 12. Transmitting component 50 is configured to receive an optical image, e.g., an image of the surgical site, and to transmit the image (schematically indicated by dotted line 52) through the elongated tubular member 12, e.g., within the fiber optic bundle, to a remote, external video display monitor (not shown). Transmitting component 50 may be coupled, at a proximal end of arthroscope 10, to an antenna (not shown) for wirelessly transmitting the image to the video display (not shown), or, alternatively, to a cable (not shown) for wired transmission of the image to the video display (not shown). Thus, in either configuration, the video display (not shown) provides a video image of the illuminated surgical site.

As can be appreciated, light source 40 illuminates the field of view, permitting transmitting component 50 to receive the optical image and transmit the image to the video display (not shown). Thus, the field of view of arthroscope 10 within the body, e.g., within the joint space, is limited to the area illuminated by light source 40. As mentioned above, the light source of arthroscope 10 is configured to direct light 42 from distal end 16 of elongated tubular member 12 distally along longitudinal axis "X." Therefore, at least initially, the field of view of arthroscope 10, i.e., the image transmitted to the video display, is limited to the illuminated area extending distally and axially from distal end 16 of arthroscope 10, i.e., the area directly in front of distal end 16 of arthroscope 10.

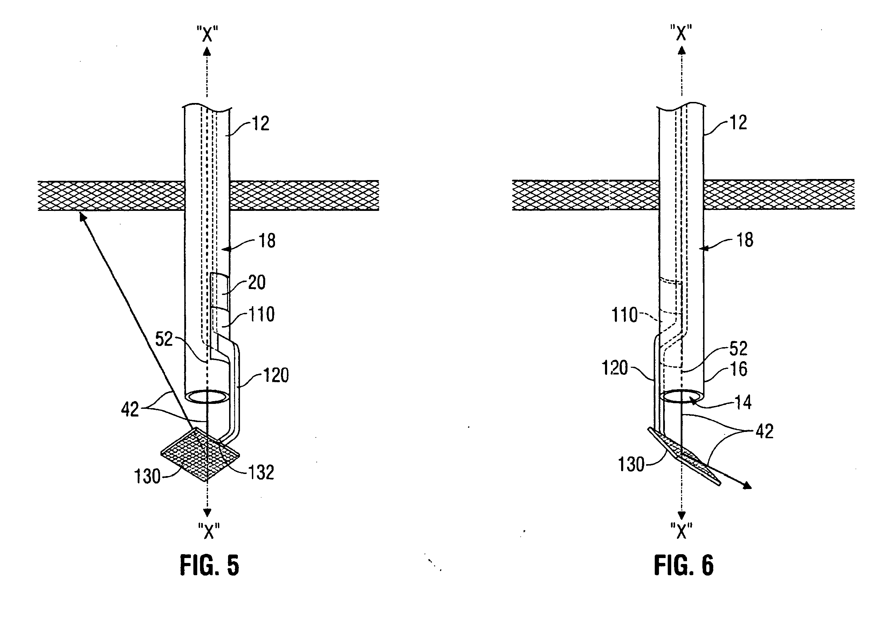

The operation of mirrored arthroscope 10, i.e., arthroscopic camera 10 including integrated mirror assembly 100 or an arthroscopic camera having a releasable mirror assembly disposed thereon, will now be described with reference to Figs. 5-6.

Initially, mirror assembly 100 is disposed in a retracted position (Fig. 1) such that mirror 130 is positioned adjacent outer surface 18 of elongated tubular member 12. It is envisioned that mirror 130 be substantially aligned with mirror assembly 100 and, more particularly, with arm 120 of mirror assembly 100 when in the retracted position (Fig. 1), such that mirror assembly 100 protrudes radially outwardly a minimum distance from elongated tubular member 12, i.e., such that arthroscope 10, including mirror assembly 100 defines a minimum diameter. As can be appreciated, with mirror assembly 100 positioned adjacent elongated tubular member 12 and protruding minimally therefrom, a width of arthroscope 10 is not substantially effected by the presence of mirror assembly 100 disposed on outer surface 18 of elongated tubular member 12.

With mirror assembly 100 of arthroscope 10 in the retracted position, as mentioned above, arthroscope 10 may be inserted through an opening in tissue and into an internal body space, e.g., the joint space. The rigidity of elongated tubular member 12 facilitates the positioning of distal end 16 of arthroscope 10 within the joint space while protecting the internal components, e.g., light source 40 and transmitting component 50, of arthroscope 10.

Once distal end 16 of arthroscope 10 is positioned within the joint space, as shown in Figs. 5-6, the internal components of arthroscope 10, e.g., the fiber optic bundle, or LED and camera, may be activated to provide a video image of the joint space. However, at this point, as mentioned above, the field of view of arthroscope 10 is limited to the illuminated area extending distally from arthroscope 10 along longitudinal axis "X." Unfortunately, manipulating arthroscope 10 within the joint space to better view a defect or other condition within the joint space may cause damage surrounding tissue or may simply be inhibited by the spatial constraints of the joint space and/or of the small opening through tissue. Even where tissue damage is not an issue, articulating and/or manipulating arthroscope 10 may only provide a somewhat larger, but still limited range of viewing directions, especially where the defect or other condition to be viewed is positioned at an angle approaching 90 degrees or greater with respect to longitudinal axis "X."

Accordingly, in order to better view the joint space, the mirror assembly 100 may be moved to the extended position to re-direct light 42 emitted from distal end 16 of arthroscope 10. For example, base 110 of mirror assembly 100 may be translated along track 20 from the proximal end 22 (Fig. 2) thereof to distal end 24 (Fig. 1) thereof such that at least a portion of arm 120 and mirror 130 extend distally beyond distal end 16 of elongated tubular member 12 of arthroscope 10. In embodiments where mirror assembly 100 is detachable from elongated tubular member 12, arm 120 may be translated along the track (not shown) defined within base 110 to similarly extend arm 120 and mirror 130 distally beyond distal end 16 of elongated tubular member 12. Transitioning mirror assembly 100 from the retracted position to the extended position may be effected, as mentioned above, by manipulating the external control member (not shown) to mechanically or electrically extend mirror assembly 100.

In the extended position, as shown in Figs. 5 and 6, and as mentioned above, mirrored surface 134 of mirror 130 extends distally from distal end 16 of elongated tubular member 12. From this position, mirror 130 may be angled with respect to arm 120 from about zero degrees, wherein mirror 130 is substantially parallel with respect to longitudinal axis "X," to at least 90 degrees, wherein mirror 130 is substantially perpendicular to longitudinal axis "X." As can be appreciated, when mirror 130 is substantially parallel with respect to longitudinal axis "X," light 42 emitted from distal end 16 of elongated tubular member 12 is undisturbed. However, as mirror 130 is angled, light 42 emitted from elongated tubular member 12 is reflected off mirrored surface 134 of mirror 130 and re-directed off the longitudinal axis "X." The control member (not shown) may be used to mechanically and/or electrically manipulate the position of mirror 130 with respect to the longitudinal axis "X."

More particularly, a user may angle mirror 130, e.g., via the control member (not shown) and control wire(s) 30, 30', to more fully view the surgical site, e.g., the joint space. For example, the user may angle mirror 130 at a relatively small angle with respect to longitudinal axis "X," such that light 40 is re-directed in a distal, but off-axis direction, as shown in Fig. 6. Alternatively, the user may angle mirror 130 at a greater angle with respect to longitudinal axis "X," to re-direct light in a substantially opposite, or proximal direction, as shown in Fig. 5. Further, arthroscope 10 may be rotated about longitudinal axis "X," as shown in Fig.6, to define even more viewing directions. Thus, as can be appreciated, mirror 130 may be angled with respect to arm 120 and/or arthroscope 10 may be rotated about its longitudinal axis "X" to provide a 360 degree view of the joint space, without the need to maneuver elongated tubular member 12 off-axis. Such a feature is advantageous in that off-axis, or radial translation and/or angling of arthroscope 10 may cause tissue damage and/or may not be practical due to the spatial constraints of the joint space. Additionally, the ability to obtain a 360 degree view of the surgical site without translating arthroscope 10 is advantageous in that arthroscope 10 may be inserted substantially perpendicularly with respect to the opening in tissue, i.e., arthroscope 10 need not be angled for a better viewing position, thereby reducing the required incision diameter. Further, due to the configuration of mirrored arthroscope 10, this 360 degree field of view is provided without the distortion common to wide angle lenses.

Prior to removal of arthroscope 10 from the joint space, when the procedure is complete, mirror assembly 100 is moved back to the retracted position, e.g., via the control member (not shown). Then, arthroscope 10, having a minimized diameter, may be removed from the opening in tissue.

It is envisioned that mirror assembly 100, along with arthroscope 10, be sterilizable such that arthroscope 10 including integrated mirror assembly 100 may be used repeatedly. On the other hand, in embodiments where mirror assembly 100 is detachable from arthroscope 10, mirror assembly 100 may be disposable. In other words, mirror assembly 100 may be discarded after a single use, and a new mirror assembly 100 may be provided, e.g., clipped onto, arthroscope 10 for each ensuing procedure to be performed.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A surgical instrument comprising:
an elongated tubular member (12) defining an outer surface (18), a longitudinal axis and having a lumen (14) extending therethrough;
a light source (40) disposed within the elongated tubular member (12) and configured to emit light from a distal end (16) of the elongated tubular member;
a mirror assembly (100) coupled to the distal end (16) of the elongated tubular member, **characterised in that** the mirror assembly comprising a base (110) engaged within a track (20) disposed on and extending longitudinally along outer surface (18) of tubular member (18);
an arm (120) fixedly engaged at a first end (122) to the base (110) and hingeably coupled to a mirror (130) at a second end thereof; and
the base (110) being translatable along the track (20) to move the mirror assembly between a retracted position, wherein the mirror (130) is aligned with the arm (120) and positioned adjacent the outer surface (18) of the elongated tubular member, and an extended position, wherein the mirror extends distally from the distal end (16) of the elongated tubular member (12) to re-direct light emitted by the light source (40).

2. The surgical instrument according to claim 1, wherein the light source (40) includes a fiber optic bundle extending through the elongated tubular member, the fiber optic bundle configured to capture and transmit an image from the distal end (16) of the elongated tubular member (12) to a remote video display for displaying the image as a video image.

3. The surgical instrument according to claim 1 or claim 2, wherein the light source (40) is a light emitting diode (LED).

4. The surgical instrument according to any preceding claim, further comprising a camera disposed within the elongated tubular member (12) and configured to capture and transmit an image from the distal end (16) of the elongated tubular member to a remote video display for displaying the image as a video image.

5. The surgical instrument according to any preceding claim, wherein the mirror (130) is hingeable with respect to the arm (120) to re-direct light emitted by the light source (40) radially off the longitudinal axis of the elongated tubular member (12) at an angle of about zero degrees to about 90 degrees in each of the distal and proximal directions.

6. The surgical instrument according to claim 5, wherein the hinging of the mirror (130) with respect to the arm (120) is one of mechanically controlled and electrically controlled.

7. The surgical instrument according to any preceding claim, wherein the mirror assembly (100) is releasably coupled to the distal end of the elongated tubular member.

8. The surgical instrument according to any preceding claim, wherein the elongated tubular member (12) is formed from a rigid material.

9. The surgical instrument according to any preceding claim, wherein the mirror (130) has one of a concave and a convex mirrored surface.

10. The surgical instrument according to any preceding claim, wherein the movement of the mirror assembly between the retracted position and the extended position is one of mechanically controlled and electrically controlled.

11. The surgical instrument according to claim 10 further comprising a control wire (30) coupled to the mirror assembly (100) at a first end thereof, the control wire configured to couple to a control member at a second end thereof for remotely controlling the hinging of the mirror with respect to the arm.

## Patentansprüche

1. Chirurgisches Instrument, umfassend:
ein längliches Röhrenglied (12), das eine Außenoberfläche (18) definiert, eine Längsachse, und das ein Lumen (14) aufweist, das sich durch es hindurch erstreckt;
eine Lichtquelle (40), die innerhalb des länglichen Röhrenglieds (12) angeordnet ist und dafür eingerichtet ist, Licht von einem distalen Ende (16) des länglichen Röhrenglieds zu emittieren;
eine Spiegelanordnung (100), die mit dem distalen Ende (16) des länglichen Röhrenglieds gekoppelt ist, **dadurch gekennzeichnet, dass** die Spiegelanordnung eine Basis (110) umfasst, die innerhalb einer Spur (20), die auf der Außenoberfläche (18) des Röhrenglieds (18) angeordnet ist und sich längs entlang ihr erstreckt, angebracht ist;
einen Arm (120), der fest an einem ersten Ende (122) der Basis (110) angebracht ist und gelenkig an einem zweiten Ende dessen mit einem Spiegel (130) gekoppelt ist; und
wobei die Basis (110) entlang der Strecke (20) translatierbar ist, um die Spiegelanordnung zwischen einer eingefahrenen Stellung, in der der Spiegel (130) mit dem Arm (120) gefluchtet ist und angrenzend an die Außenoberfläche (18) des länglichen Röhrenglieds angeordnet ist, und einer ausgefahrenen Stellung, in der der Spiegel sich distal von dem distalen Ende (16) des länglichen Röhrenglieds (12) erstreckt, um Licht, das von der Lichtquelle (40) emittiert wird, umzuleiten, zu bewegen.

2. Chirurgisches Instrument gemäß Anspruch 1, bei der die Lichtquelle (40) ein faseroptisches Bündel beinhaltet, das sich durch das längliche Röhrenglied erstreckt, wobei das faseroptische Bündel dafür eingerichtet ist, ein Bild von dem distalen Ende (16) des länglichen Röhrenglieds (12) aufzunehmen und an eine sich entfernt befindende Videoanzeige zu übertragen, um das Bild als ein Videobild anzuzeigen.

3. Chirurgisches Instrument gemäß Anspruch 1 oder Anspruch 2, bei dem die Lichtquelle (40) eine Licht emittierende Diode (LED) ist.

4. Chirurgisches Instrument gemäß einem der vorhergehenden Ansprüche, das außerdem eine Kamera umfasst, die innerhalb des länglichen Röhrenglieds (12) angeordnet ist und dafür eingerichtet ist, ein Bild von dem distalen Ende (16) des länglichen Röhrenglieds aufzunehmen und an eine sich entfernt befindende Videoanzeige zu übertragen, um das Bild als ein Videobild anzuzeigen.

5. Chirurgisches Instrument gemäß einem der vorhergehenden Ansprüche, bei dem der Spiegel (130) bezüglich des Arms (120) gelenkig ist, um Licht, das durch die Lichtquelle (40) emittiert wird, radial von der Längsachse des länglichen Röhrenglieds (12) um einen Winkel von ungefähr null Grad bis ungefähr 90 Grad in jede der distalen und proximalen Richtungen umzuleiten.

6. Chirurgisches Instrument gemäß Anspruch 5, bei dem das Gelenk des Spiegels (130) bezüglich des Arms (120) eines ist von mechanisch gesteuert und elektrisch gesteuert.

7. Chirurgisches Instrument gemäß einem der vorhergehenden Ansprüche, bei dem die Spiegelanordnung (100) lösbar mit dem distalen Ende des länglichen Röhrenglieds gekoppelt ist.

8. Chirurgisches Instrument gemäß einem der vorhergehenden Ansprüche, bei dem das längliche Röhrenglied (12) aus einem starren Werkstoff gebildet ist.

9. Chirurgisches Instrument gemäß einem der vorhergehenden Ansprüche, bei dem der Spiegel (130) eine von einer konkaven und einer konvexen verspiegelten Oberfläche aufweist.

10. Chirurgisches Instrument gemäß einem der vorhergehenden Ansprüche, bei dem die Bewegung der Spiegelanordnung zwischen der eingefahrenen Stellung und der ausgefahrenen Stellung eines ist von mechanisch gesteuert und elektrisch gesteuert.

11. Chirurgisches Instrument gemäß Anspruch 10, das außerdem einen Steuerdraht (30) umfasst, der mit der Spiegelanordnung (100) an einem ersten Ende dessen gekoppelt ist, wobei der Steuerdraht dafür eingerichtet ist, mit einem Steuerglied an einem zweiten Ende dessen zu koppeln, um das Gelenk des Spiegels bezüglich des Arms aus der Ferne zu steuern.

## Revendications

1. Instrument chirurgical comprenant :
un élément tubulaire allongé (12) définissant une surface externe (18), un axe longitudinal et ayant une lumière (14) s'étendant à travers celui-ci ;
une source de lumière (40) disposée à l'intérieur de l'élément tubulaire allongé (12) et configurée pour émettre de la lumière à partir d'une extrémité distale (16) de l'élément tubulaire allongé ;
un ensemble miroir (100) couplé à l'extrémité distale (16) de l'élément tubulaire allongé, **caractérisé en ce que** l'ensemble miroir comprenant une base (110) insérée à l'intérieur d'un rail (20) disposé sur et s'étendant longitudinalement le long d'une surface externe (18) d'un élément tubulaire (18) ;
un bras (120) en prise de manière fixe à une première extrémité (122) avec la base (110) et couplé de manière articulée à un miroir (130) à une seconde extrémité de celui-ci ; et
la base (110) pouvant être translatée le long du rail (20) pour déplacer l'ensemble miroir entre une position rétractée, dans lequel le miroir (130) est aligné avec le bras (120) et positionné de manière adjacente à la surface externe (18) de l'élément tubulaire allongé, et une position étendue, dans lequel le miroir s'étend distalement à partir de l'extrémité distale (16) de l'élément tubulaire allongé (12) pour rediriger la lumière émise par la source de lumière (40).

2. Instrument chirurgical selon la revendication 1, dans lequel la source de lumière (40) comprend un faisceau de fibres optiques s'étendant à travers l'élément tubulaire allongé, le faisceau de fibres optiques étant configuré pour capturer et transmettre une image de l'extrémité distale (16) de l'élément tubulaire allongé (12) à un affichage vidéo distant pour afficher l'image sous la forme d'une image vidéo.

3. Instrument chirurgical selon la revendication 1 ou la revendication 2, dans lequel la source de lumière (40) est une diode électroluminescente (DEL).

4. Instrument chirurgical selon l'une quelconque des revendications précédentes, comprenant en outre une caméra disposée à l'intérieur de l'élément tubulaire allongé (12) et configurée pour capturer et transmettre une image de l'extrémité distale (16) de l'élément tubulaire allongé à un affichage vidéo distant pour l'affichage de l'image sous la forme d'une image vidéo.

5. Instrument chirurgical selon l'une quelconque des revendications précédentes, dans lequel le miroir (130) peut être articulé par rapport au bras (120) pour rediriger la lumière émise par la source de lumière (40) radialement à l'écart de l'axe longitudinal de l'élément tubulaire allongé (12) à un angle d'environ zéro degré à environ 90 degrés dans chacune des directions distale et proximale.

6. Instrument chirurgical selon la revendication 5, dans lequel l'articulation du miroir (130) par rapport au bras (120) est l'une parmi une articulation commandée mécaniquement et une articulation commandée électriquement.

7. Instrument chirurgical selon l'une quelconque des revendications précédentes, dans lequel l'ensemble miroir (100) est couplé de manière détachable à l'extrémité distale de l'élément tubulaire allongé.

8. Instrument chirurgical selon l'une quelconque des revendications précédentes, dans lequel l'élément tubulaire allongé (12) est formé à partir d'un matériau rigide.

9. Instrument chirurgical selon l'une quelconque des revendications précédentes, dans lequel le miroir (130) possède l'une d'entre une surface réfléchissante concave et une surface réfléchissante convexe.

10. Instrument chirurgical selon l'une quelconque des revendications précédentes, dans lequel le déplacement de l'ensemble miroir entre la position rétractée et la position étendue est l'un parmi un déplacement commandé mécaniquement et un déplacement commandé électriquement.

11. Instrument chirurgical selon la revendication 10, comprenant en outre un fil de commande (30) couplé à l'ensemble miroir (100) à une première extrémité de celui-ci, le fil de commande étant configuré pour être couplé à un élément de commande à une seconde extrémité de celui-ci pour commander à distance l'articulation du miroir par rapport au bras.
